# EUROPÄISCHE PATENTSCHRIFT

(11) **EP 1 094 888 B1**
(45) Veröffentlichungstag und Bekanntmachung des Hinweises auf die Patenterteilung: **08.03.2006**
(21) Anmeldenummer: 99931076.6
(22) Anmeldetag: 16.06.1999
(51) Int. Cl.: B01F 17/00

(54) **FLIESSFÄHIGE ZUBEREITUNGEN VOM EMULSIONSTYP W/O MIT ERHÖHTEM WASSERGEHALT**
FLOWABLE PREPARATIONS OF THE WATER-IN-OIL EMULSION TYPE HAVING AN INCREASED WATER CONTENT
PREPARATIONS COULANTES DE TYPE EMULSION EAU DANS L'HUILE A TENEUR EN EAU AUGMENTEE

(30) Priorität: 18.06.1998 DE 19827184; 04.11.1998 DE 19850768
(43) Veröffentlichungstag der Anmeldung: 02.05.2001
(73) Patentinhaber: Beiersdorf Aktiengesellschaft, 20245 Hamburg (DE)
(72) Erfinder: BLECKMANN, Andreas, D-22926 Ahrensburg (DE); KRÖPKE, Rainer, D-22869 Schenefeld (DE); SCHNEIDER, Günther, D-22607 Hamburg (DE)
(86) Internationale Anmeldenummer: PCT/EP1999/004164
(87) Internationale Veröffentlichungsnummer: WO 1999/065598

(56) Entgegenhaltungen:
- WO-A-98/17232
- WO-A-98/17238

## Beschreibung

Die vorliegende Erfindung betrifft kosmetische und dermatologische Zubereitungen, insbesondere solche vom Typ Wasser-in-Öl, Verfahren zu ihrer Herstellung sowie ihre Verwendung für kosmetische und medizinische Zwecke.

Die menschliche Haut übt als größtes Organ des Menschen zahlreiche lebenswichtige Funktionen aus. Mit durchschittlich etwa 2 m² Oberfläche beim Erwachsenen kommt ihr eine herausragende Rolle als Schutz- und Sinnesorgan zu. Aufgabe dieses Organs ist es, mechanische, thermische, aktinische, chemische und biologische Reize zu vermitteln und abzuwehren. Außerdem kommt ihr eine bedeutende Rolle als Regulations- und Zielorgan im menschlichen Stoffwechsel zu.

Unter kosmetischer Hautpflege ist in erster Linie zu verstehen, die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) zu stärken oder wiederherzustellen sowie ihre Homschicht bei aufgetretenen Schäden in ihrem natürlichen Regenerationsvermögen zu unterstützen.

Werden die Barriereeigenschaften der Haut gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und Öl und liegen Öltröpfchen fein verteilt in Wasser vor, so handelt es sich um eine ÖI-in-Wasser-Emulsion (O/W Emulsion, z. B. Milch). Der Grundcharakter einer O/W-Emulsion ist durch das Wasser geprägt. Bei einer Wasser-in-ÖI-Emulsion (W/O-Emulsion, z. B. Butter) handelt es sich um das umgekehrte Prinzip, wobei der Grundcharakter hier durch das Öl bestimmt wird.

Natürlich ist dem Fachmann eine Vielzahl von Möglichkeiten bekannt, stabile W/O-Zubereitungen zur kosmetischen oder dermatologischen Anwendung zu formulieren, beispielsweise in Form von Cremes und Salben, die im Bereich von Raum- bis Hauttemperatur streichfähig sind, oder als Lotionen und Milche, die in diesem Temperaturbereich eher fließfähig sind. Der Stand der Technik kennt allerdings nur wenige Formulierungen, die so dünnflüssig sind, daß sie beispielsweise sprühbar wären.

Zudem haben dünnflüssige Zubereitungen des Standes der Technik häufig den Nachteil, daß sie instabil, auf einen engen Anwendungsbereich oder eine begrenzte Einsatzstoffauswahl begrenzt sind. Dünnflüssige Produkte, in denen beispielsweise stark polare Öle - wie die in handelsüblichen Produkten sonst häufig verwendeten Pflanzenöle - ausreichend stabilisiert sind, gibt es daher zur Zeit auf dem Markt nicht.

W/O-Emulsionen mit hohem Wassergehalt und einer geringen Viskosität, die darüberhinaus eine Lagerstabilität aufweisen, wie sie für marktgängige Produkte gefordert wird, sind nach dem Stand der Technik nur sehr aufwendig zu formulieren. Dementsprechend ist das Angebot an derartigen Formulierungen äußerst gering. Gleichwohl könnten derartige Formulierungen dem Verbraucher bisher nicht gekannte kosmetische Leistungen bieten.

Eine Aufgabe der vorliegenden Erfindung war es, Zubereitungen zur Verfügung zu stellen, welche eine sehr geringe Viskosität haben und nicht die Nachteile des Standes der Technik aufweisen.

Eine weitere Aufgabe der vorliegenden Erfindung war es. Zubereitungen zur Verfügung zustelten, welche mit einem hohen Gehalt an wasserlöslichen und/oder wassermischbaren Substanzen mit kosmetischer oder dermatologischer Wirksamkeit beladen werden können, ohne daß die galenische Qualität oder andere Eigenschaften der Zubereitungen beeinträchtigt wären.

Als sogenannte "High Internal Phase"- Emulsionen werden nach K.J.Lissant: *The Geometry of High-Internal-Phase-Ratio Emulsions;* Journal of Colloid and Interface Science **22**, 462-468 (1966) Emulsionen mit einer inneren Phase von mehr als 70 % definiert. Die Herstellung stabiler, fließfähiger Wasser-in-Öl-Emuisionen mit einem Wassergehalt von mehr als 70% stellt sich als sehr schwierig dar. Insbesondere sind "High Internal Phase"-W/O-Emulsionen mit einem sehr hohen Wassergehalt von mehr als 85% ("Very High Internal Phase"-W/O-Emulsionen) nicht zugänglich.

Die üblicherweise bei Wasser-in-Öl-Emulsionen angewandte Technik der Variation des Phasen-Volumen-Verhältnisses (d.h. Einarbeitung höherer Mengen an flüssigen Lipiden) kann, auf Grund des niedrigen Lipidanteils bei "High Internal Phase"-W/O-Emulsionen nur bedingt, bei "Very High Internal Phase"-W/O-Emulsionen) überhaupt nicht genutzt werden. Es sind daher nur Wasser-in-Öl-Emulsionen mit einer festen bis halbfesten Konsistenz zugänglich. Auch die Verwendung von polaren Lipiden, durch die üblicherweise niedrigviskosere Wasser-in-Öl-Emulsionen erhalten werden, führt nicht zum gewünschten Erfolg.

Überraschend hat sich gezeigt, daß Wasser-in-Öl-Emulsionen
(a) einer Viskosität von höchstens 5.000 mPa·s
(b) eines Gehaltes an Wasser und gegebenfalls wasserlöslichen Substanzen von insgesamt mindestens 75 Gew.%, und eines Gehaltes an Lipiden, Emulgatoren und lipophilen Bestandteilen von insgesamt höchstens 15%, jeweils bezogen auf das Gesamtgewicht der Zubereitungen.
(c) deren Ölphase wenigstens 75 Gew.% eine oder mehrere Substanzen umfaßt, gewählt aus der Gruppe der
   - bei Raumtemperatur flüssigen, unpolaren Lipiden, welche eine Polarität größer als 30 mN/m aufweisen besteht, und/oder
   - der Siliconöle beliebiger Polarität

   wobei dieser Gewichtsanteil bezogen ist auf das Gesamtgewicht der Ölphase,
(d) enthaltend wenigstens eine grenzflächenaktive Substanz, gewählt aus der Gruppe der Substanzen der der allgemeinen Formel (I) 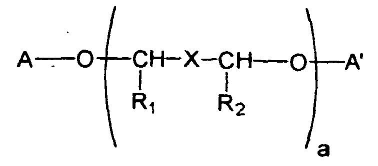
   - wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema 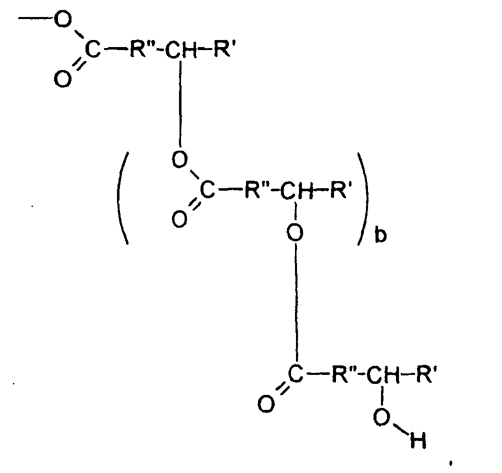
      wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,
   - a eine Zahl von 1 bis 100, vorzugsweise 2 bis 60, insbesondere 5 bis 40 darstellt,
   - X eine Einfachbindung oder die Gruppe 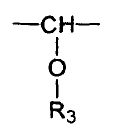
   - darstellt,
   - R₁ und R₂ unabhängig voneinander aus der Gruppe H, Methyl gewählt werden,
   - R₃ gewählt wird aus der Gruppe H, sowie der verzweigten und unverzweigten, gesättigten und ungesättigten Alkyl- und Acylreste mit 1 - 20 Kohlenstoffatomen,

   den Nachteilen des Standes der Technik abhelfen.

Die Strukturformel ist nicht so zu interpretieren, daß durch den Index a alle in der Klammer repräsentierten Reste R₁, R₂ bzw R₃ im gesamten Molekül jeweils gleich sein müssen. Vielmehr können diese Reste in jedem der a Fragmente 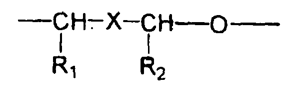
frei gewählt werden.

Es ist möglich und vorteilhaft, den Gesamtgehalt an Wasser und wasserlöslichen Substanzen der erfindungsgemäßen W/O-Emulsionen auf größer als 80 Gew.-%, insbesondere größer als 85 Gew.% zu wählen, jeweils bezogen auf das Gesamtgewicht der Zubereitungen.

Ein Beispiel für besonders vorteilhaft im Sinne der vorliegenden Erfindung zu verwendende grenzflächenaktiven Substanzen ist das Polyethylenglycol-30-Dipolyhydroxystearat (PEG-30-Dipolyhydroxystearat), welches von der Gesellschaft ICI Surfactants unter der Warenbezeichnung ARLACEL® P135 verkauft wird.

Die Gesamtmenge an den erfindungsgemäß verwendeten grenzflächenaktiven Substanzen in den fertigen kosmetischen oder dermatologischen Zubereitungen wird vorteilhaft aus dem Bereich von 0,1 - 30 Gew.%, bevorzugt 0,25 - 5,0 Gew.-% insbesondere 0,75 - 3,5 Gew.% gewählt, bezogen auf das Gesamtgewicht der Zubereitungen.

Zwar ist bekannt, daß sich mit Emulgatoren der vorab beschriebenen Art W/O-Emulsionen mit hohem Wassergehalt erzeugen lassen. Dennoch konnte der bekannte Stand der Technik nicht den Weg zur vorliegenden Erfindung weisen.

Im Rahmen der vorliegenden Offenbarung wird als Oberbegriff für Fette, Öle, Wachse und dergleichen gelegentlich der Ausdruck "Lipide" verwendet, wie dem Fachmanne durchaus geläufig ist. Auch werden die Begriffe "Ölphase" und "Lipidphase" synonym angewandt.

Öle und Fette unterscheiden sich unter anderem in ihrer Polarität, welche schwierig zu definieren ist. Es wurde bereits vorgeschlagen, die Grenzflächenspannung gegenüber Wasser als Maß für den Polaritätsindex eines Öls bzw. einer Ölphase anzunehmen. Dabei gilt, daß die Polarität der betreffenden Ölphase umso größer ist, je niedriger die Grenzflächenspannung zwischen dieser Ölphase und Wasser ist. Erfindungsgemäß wird die Grenzflächenspannung als ein mögliches Maß für die Polarität einer gegebenen Ölkomponente angesehen.

Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen.

Als Grenze, unterhalb derer eine Ölphase als "polar" und oberhalb derer eine Ölphase als "unpolar" gilt, werden erfindungsgemäß 30 mN/m angesehen.

Als besonders vorteilhaft haben sich folgende, bei Raumtemperatur, flüssige unpolare Lipide erwiesen: Kohlenwassersoffe (Mineralöle, Cyloparaffin, Polyisobutene, Polydecene), nicht ethoxylierte bzw. propopoxylierte Ether (Caprylylether / Cetiol OE) sowie Silikonöle (Dimethicone, Cylomethicone, Dimethiconol).

Nach obiger Definition der Polarität gelten Silikonöle nicht als unpolar sondern fallen in der Regel in die mittelpolare Region (typischerweise zwischen 20 und 30 mN/m).

Es ist erfindungsgemäß möglich, einen gewissen Anteil polarer Lipide in der Lipidmischung zu dulden, keinesfalls soll dieser Anteil jedoch 25 Gew.% übersteigen, beträgt bevorzugt weniger als 15 Gew.-% und soll im Idealfalle nicht mehr als < 10 Gew.-%, bezogen auf die Gesamtlipidphase, betragen.

Gemäß der hiermit vorgelegten Lehre sind W/O-Emulsionen erhältlich, deren Viskosität bei 25° C kleiner als 5.000 mPa·s (= Millipascalsekunden) insbesondere kleiner als 4.000 mPa·s, bevorzugt kleiner als 3.500 mPa·s (HAAKE Viscotester VT-02).

Vorteilhaft werden die erfindungsgemäßen Öle ebenfalls gewählt aus der Gruppe der Paraffinöle, Polyolefine sowie Vaseline (Petrolatum). Unter den Polyolefinen sind Polydecene und hydriertes Polyisobuten die bevorzugten Substanzen.

Die Ölphase kann im Sinne der vorliegenden Erfindung ferner - sofern die in den Patentansprüchen aufgeführten Merkmale beachtet werden - vorteilhaft Substanzen enthalten, gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropytmyristat, Isopropylpalmitat, Isopropylstearat, Isopropyloleat, n-Butylstearat, n-Hexyllaurat, n-Decyloleat, Isooctylstearat, Isononylstearat. Isononylisononanoat, 2-Ethylhexylpalmitat, 2-Ethylhexyllaurat, 2-Hexyldecylstearat, 2-Octyldodecylpalmitat, Oleytoleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkemöl und dergleichen mehr.

Gewünschtenfalls können in der Ölphase einzusetzende Fett- und/oder Wachskomponenten - als Nebenbestandteile in geringerer Menge - aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montanwachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), Syncrowax HGLC (C₁₆₋₃₆ -Fettsäuretriglycerid) und Syncrowax AW 1C (C₈₋₃₆-Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ - Alkyl Bienenwachs). Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin. Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan.

Gewünschtenfalls können die Fett- und/oder Wachskomponenten sowohl einzeln als auch im Gemisch vorliegen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als Lipidkomponente der Ölphase einzusetzen.

Von den Kohlenwasserstoffen sind Paraffinöl, hydrierte Polyolefine (z.B. hydriertes Polyisobuten) Squalan und Squalen vorteilhaft im Sinne der vorliegenden Erfindung zu verwenden.

Erfindugsgemäß besonders vorteilhaft sind solche Emulsionen, die dadurch gekennzeichnet sind, daß die Ölphase zu mindestens 50 Gew.-%, bevorzugt zu mehr als 75 Gew.-% aus mindestens einer Substanz, gewählt aus der Gruppe gewählt aus der Gruppe Vaseline (Petrolatum), Paraffinöl und Polyolefine, unter den letzteren bevorzugt: Polydecenen, besteht.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft kann Cyclomethicon (Octamethylcyclotetrasiloxan) eingesetzt werden. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol. 1,2-Propandiot. Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z:B. Hyaluronsäure, Xanthangummi, Hydroxypropylmethylcellutose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, oder auch der Typen ETD (Easy-to-disperse) 2001, 2020, 2050, jeweils einzeln oder in beliebigen Kombinationen untereinander.

Ein besonderer Vorzug der vorliegenden Erfindung ist es, daß sie gestattet, hohe Konzentrationen an Polyolen, insbesondere Glycerin einzusetzen.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Ψ-Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γγ-Linoleyl-, Cholesteryl - und Glycerylester) sowie deren Salze, Dilaurylthiodipropionat. Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA. EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können öllösliche Antioxidantien eingesetzt werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei bevorzugte Wirkstoffe Antioxidantien sind, welche die Haut vor oxidativer Beanspruchung schützen können. Bevorzugte Antioxidantien sind dabei Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmann natürlich bekannt, daß kosmetische Zubereitungen zumeist nicht ohne die üblichen Hilfs- und Zusatzstoffe denkbar sind. Die erfindungsgemäßen kosmetischen und dermatologischen Zubereitungen können dementsprechend ferner kosmetische Hilfsstoffe enthalten, wie sie üblicherweise in solchen Zubereitungen verwendet werden, beispielsweise Konsistenzgeber, Stabilisatoren, Füllstoffe, Konservierungsmittel, Parfüme, Substanzen zum Verhindern des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, entzündungshemmende Substanzen, zusätzliche Wirkstoffe wie Vitamine oder Proteine, Lichtschutzmittel, Insektenrepellentien, Bakterizide, Viruzide, Wasser, Salze, antimikrobiell, proteolytisch oder keratolytisch wirksame Substanzen, Medikamente oder andere übliche Bestandteile einer kosmetischen oder dermatologischen Formulierung wie Alkohole, Polyole, Polymere, Schaumstabilisatoren, organische Lösungsmittel oder auch Elektrolyte.

Letztere können beispielsweise gewählt werden aus der Gruppe der Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte sind vorteilhaft, z.B. Lactate, Acetate, Benzoate, Propionate, Tartrate, Citrate, Aminosäuren, Ethylendiamintetraessigsäure und deren Salze und andere mehr. Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Besonders bevorzugt sind Kaliumchlorid, Kochsalz, Magnesiumsulfat, Zinksulfat und Mischungen daraus.

Mutatis mutandis gelten entsprechende Anforderungen an die Formulierung medizinischer Zubereitungen.

Die erfindungsgemäßen W/O-Emulsionen können als Grundlage für kosmetische oder dermatologische Formulierungen dienen. Diese können wie üblich zusammengesetzt sein und beispielsweise zur Behandlung und der Pflege der Haut und/oder der Haare, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika oder Dermatika üblichen Weise auf die Haut und/oder die Haare in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcrème, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Die dünnflüssigen kosmetischen oder dermatologischen Mittel gemäß der Erfindung können beispielsweise als aus Aerosolbehältern, Quetschflaschen oder durch eine Pumpvorrichtung versprühbare Präparate vorliegen oder in Form einer mittels Roll-on-Vorrichtungen auftragbaren flüssigen Zusammensetzung, jedoch auch in Form einer aus normalen Flaschen und Behältern auftragbaren Emulsion.

Als Treibmittel für aus Aerosolbehäftern versprühbare kosmetische oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescrèmes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure-(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester. Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- Derivate des 1,3,5-Triazins, vorzugsweise 2,4,6-Trianitino-(p-carbo-2'-ethyl-1'-hexyloxy) -1,3,5-triazin.

Die Liste der genannten UVB-Filter, die in Kombination mit den erfindungsgemäßen Wirkstoffkombinationen verwendet werden können, soll selbstverständlich nicht limitierend sein.

Es kann auch von Vorteil sein, erfindungsgemäße Lipodispersionen mit UVA-Fittern zu formulieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion.

Erfindungsgemäße kosmetische und dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Als weitere Bestandteile können verwendet werden:
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Erfindungsgemäße Zubereitungen können auch Wirkstoffe (eine oder mehrere Verbindungen) enthalten, welche gewählt werden aus der Gruppe: Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linotensäure. Ölsäure, Eicosapentaensäure. Docosahexaensäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkemöl, Fischöle, Lebertran aber auch Ceramide und ceramidähnliche Verbindungen und so weiter. Vorteilhaft ist es auch, die Wirkstoffe aus der Gruppe der rückfettenden Substanzen zu wählen, beispielsweise Purcellinöl, Eucerit® und Neocerit®.

Die Menge solcher Wirkstoffe (eine oder mehrere Verbindungen) in den Zubereitungen gemäß der Erfindung beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiel 1 (W/O-Lotion):

| | Gew.-% |
|---|---|
| PEG-30-Dipolyhydroxystearat | 2,00 |
| Isohexadecan | 4,50 |
| Paraffinum liquidum | 4,50 |
| Glycerin | 3,00 |
| Magnesiumsulfat | 0,70 |
| Parfüm, Konservierungsmittel, Farbstoffe, Antioxidantien | q.s. |
| Wasser | ad 100,00 |

### Beispiel 2 (W/O-Lotion):

| | Gew.-% |
|---|---|
| PEG-30-Dipolyhydroxystearat | 2,00 |
| Squalan | 3,00 |
| Paraffinum liquidum | 3,00 |
| Hydriertes Polysiobuten | 3,00 |
| Glycerin | 3,00 |
| Magnesiumsulfat | 0,70 |
| Parfüm, Konservierungsmittel, Farbstoffe, Antioxidantien | q.s. |
| Wasser | ad 100,00 |

### Beispiel 3 (W/O-Lotion):

| | Gew.-% |
|---|---|
| PEG-30-Dipolyhydroxystearat | 2,00 |
| Cyloparaffin | 4,50 |
| Paraffinum liquidum | 4,50 |
| Tocopherolacetat | 0.50 |
| Glycerin | 3,00 |
| Panthenol | 0,30 |
| 1,3-Butylenglycol | 1,00 |
| Serine | 0,30 |
| Biotin | 0,10 |
| Distärkephosphat | 1,00 |
| Magnesiumsulfat | 0,70 |
| Parfüm, Konservierungsmittel, Farbstoffe, Antioxidantien | q.s. |
| Wasser | ad 100,00 |

### Beispiel 4 (W/O-Lotion):

| | Gew.-% |
|---|---|
| PEG-30-Dipolyhydroxystearat | 2,00 |
| lsohexadecan | 2,00 |
| Paraffinum subliquidum | 2,00 |
| 4-(tert.-Butyl)-4'-methoxydibenzoylmethan | 1,00 |
| Octylmethoxycinnamate | 1,50 |
| 4-Methyl-Benzylidencampher | 1,50 |
| Tris-[anilino-(p-carbo-2'-ethyl-1'-hexyloxy)]-triazin | 0,50 |
| Titandioxid | 1,00 |
| Zinkoxid | 1,00 |
| Glycerin | 1,00 |
| Magnesium Sulfate | 0,70 |
| Parfüm, Konservierungsmittel, Farbstoffe, Antioxidantien | q.s. |
| Wasser | ad 100,00 |

### Beispiel 5 (W/O-Lotion):

| | Gew.-% |
|---|---|
| PEG-30-Dipolyhydroxystearat | 2,00 |
| Isohexadecan | 4,50 |
| Paraffinum liquidum | 4,50 |
| Glycerin | 3,00 |
| Natriumchlorid | 0,70 |
| Parfüm, Konservierungsmittel, Farbstoffe, Antioxidantien | q.s. |
| Wasser | ad 100,00 |

### Beispiel 6 (flüssiges Emulsions-Make-up):

| | Gew.-% |
|---|---|
| PEG-30-Dipolyhydroxystearat | 2,00 |
| lsohexadecan | 4,50 |
| Paraffinum liquidum | 4,50 |
| Glycerin | 1,50 |
| Magnesiumsilikat | 0,50 |
| Glimmer | 0,50 |
| Eisenoxide | 0,50 |
| Titandioxid | 0,50 |
| Talkum | 0,50 |
| Magnesiumsilikat | 0,50 |
| Magnesiumsulfat | 0,70 |
| Parfüm, Konservierungsmittel, Farbstoffe, Antioxidantien | q.s. |
| Wasser | ad 100,00 |

### Beispiel 7 (W/O-Lotion):

| | Gew.-% |
|---|---|
| PEG-30-Dipolyhydroxystearat | 2,50 |
| Squalan | 3,00 |
| Polydecen | 3,00 |
| Paraffinum liquidum | 4,00 |
| Hydriertes Polysiobuten | 1,00 |
| Glycerin | 1,00 |
| Sorbitol | 5,00 |
| Magnesiumsulfat | 0,70 |
| Parfüm, Konservierungsmittel, Farbstoffe, Antioxidantien | q.s. |
| Wasser | ad 100,00 |

### Beispiel 8 (W/O-Lotion):

| | Gew.-% |
|---|---|
| PEG-30-Dipolyhydroxystearat | 2,0 |
| Isohexadecan | 4,5 |
| Paraffinum subliquidum | 4,5 |
| Glycerin | 20,0 |
| Magnesiumsulfat | 0,70 |
| Parfüm, Konservierungsmittel, Farbstoffe, Antioxidantien | q.s. |
| Wasser | ad 100,00 |

## Patentansprüche

1. Wasser-in-ÖI-Emulsionen
(a) einer Viskosität von höchstens 5.000 mPa·s
(b) eines Gehaltes an Wasser und wasserlöslichen Substanzen von insgesamt mindestens 75 Gew.%, und eines Gehaltes an Lipiden, Emulgatoren und lipophilen Bestandteilen von insgesamt höchstens 15%, jeweils bezogen auf das Gesamtgewicht der Zubereitungen.
(c) deren Ölphase wenigstens 75 Gew.% eine oder mehrere Substanzen umfaßt, gewählt aus der Gruppe der
- bei Raumtemperatur flüssigen, unpolaren Lipiden, welche eine Polarität größer als 30 mN/m aufweisen besteht, und/oder
- der Siliconöle beliebiger Polarität
wobei dieser Gewichtsanteil bezogen ist auf das Gesamtgewicht der Ölphase,
(d) enthaltend wenigstens eine grenzflächenaktive Substanz, gewählt aus der Gruppe der Substanzen der der allgemeinen Formel (I) 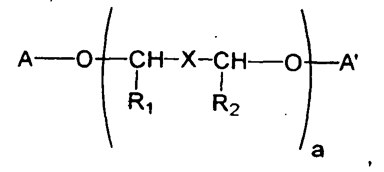
- wobei A und A' gleiche oder verschiedene organische Reste, gewählt aus der Gruppe der verzweigten und unverzweigten, gesättigten und ungesättigten Alkyl- und Acylreste und Hydroxyacylreste mit 10 - 30 Kohlenstoffatomen sowie ferner aus der Gruppe der über Esterfunktionen miteinander verbundenen Hydroxyacylgruppen, nach dem Schema 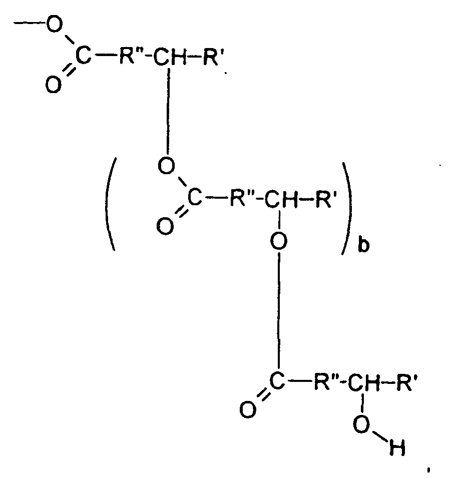
wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann,
- a eine Zahl von 1 bis 100, vorzugsweise 2 bis 60, insbesondere 5 bis 40 darstellt,
- X eine Einfachbindung oder die Gruppe 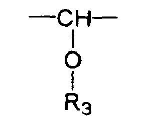
- darstellt,
- R₁ und R₂ unabhängig voneinander aus der Gruppe H, Methyl gewählt werden,
- R₃ gewählt wird aus der Gruppe H, sowie der verzweigten und unverzweigten, gesättigten und ungesättigten Alkyl- und Acylreste mit 1 - 20 Kohlenstoffatomen.

2. Emulsionen nach Anspruch 1, **dadurch gekennzeichnet, daß** ihr Gehalt an Wasser und wasserlöslichen Substanzen größer ist als 80 Gew.%, insbesondere größer als 85 Gew.-%, jeweils bezogen auf das Gesamtgewicht der Zubereitungen.

3. Emulsionen nach Anspruch 1, **dadurch gekennzeichnet, daß** als grenzflächenaktive Substanz das Polyethylenglycol-30-Dipolyhydroxystearat gewählt wird.

4. Emulsionen nach Anspruch 1, **dadurch gekennzeichnet, daß** die Ölphase zu mindestens 50 Gew.-%, bevorzugt zu mehr als 75 Gew.-% aus mindestens einer Substanz, gewählt aus der Gruppe gewählt aus der Gruppe Vaseline (Petrolatum), Paraffinöl und Polyolefine, unter den letzteren bevorzugt: Polydecenen, besteht.

## Claims

1. Water-in-oil emulsions
(a) with a viscosity of at most 5000 mPa·s
(b) with a content of water and water-soluble substances totalling at least 75% by weight, and with a content of lipids, emulsifiers and lipophilic constituents totalling at most 15%, based in each case on the total weight of the preparations,
(c) the oil phase of which comprises at least 75% of one or more substances chosen from the group of
- nonpolar lipids which are liquid at room temperature and have consists a polarity of greater than 30 mN/m, and/or
- silicones of any polarity
this weight proportion being based on the total weight of the oil phase,
(d) comprising at least one interface-active substance chosen from the group of substances of the general formula (I) 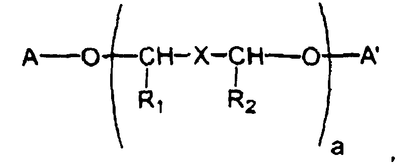
- where A and A' are identical or different organic radicals chosen from the group of branched and unbranched, saturated and unsaturated alkyl and acyl radicals and hydroxyacyl radicals having 10-30 carbon atoms, and also from the group of hydroxyacyl groups connected to one another via ester functions, in accordance with the scheme 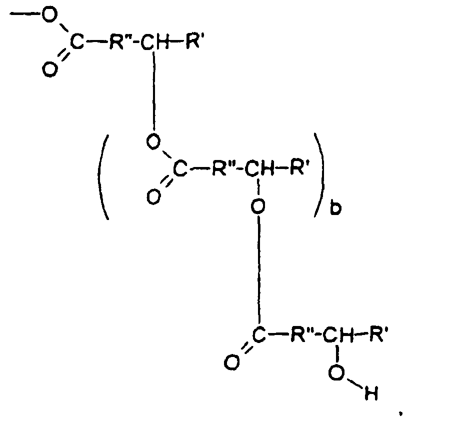
where R' is chosen from the group of branched and unbranched alkyl groups having 1 to 20 carbon atoms and R" is chosen from the group of branched and unbranched alkylene groups having 1 to 20 carbon atoms, and b can assume numbers from 0 to 200,
- a is a number from 1 to 100, preferably 2 to 60, in particular 5 to 40,
- X is a single bond or the group 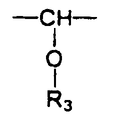
- R₁ and R₂ independently of one another are chosen from the group consisting of H and methyl,
- R₃ is chosen from the group consisting of H, and the branched and unbranched, saturated and unsaturated alkyl and acyl radicals having 1 to 20 carbon atoms.

2. Emulsions according to Claim 1, **characterized in that** their content of water and water-soluble substances is greater than 80% by weight, in particular greater than 85% by weight, in each case based on the total weight of the preparations.

3. Emulsions according to Claim 1, **characterized in that** the interface-active substance chosen is polyethylene glycol-30-dipolyhydroxystearate.

4. Emulsions according to Claim 1, **characterized in that** the oil phase consists of at least 50% by weight, preferably of more than 75% by weight, of at least one substance chosen from the group chosen from the group consisting of vaseline (petrolatum), paraffin oil and polyolefins, of the latter preferably: polydecenes.

## Revendications

1. Emulsions d'eau dans l'huile:
a) présentant une viscosité maximale de 5000 mPa.s,
b) présentant une teneur en eau et en substances solubles dans l'eau globalement d'au moins 75% en poids et une teneur globale en lipides, émulsifiants et composants lipophiles qui ne dépasse pas 15%, chaque fois par rapport au poids total des préparations,
c) dont la phase huileuse contient au moins 75% en poids d'une ou de plusieurs substances sélectionnées dans l'ensemble constitué:
- des lipides non polaires liquides à température ambiante et dont la polarité est supérieure à 30 mN/m et/ou
- des huiles de silicone de polarité quelconque,
cette proportion pondérale étant calculée par rapport au poids total de la phase huileuse,
d) qui contiennent au moins une substance tensioactive sélectionnée dans le groupe de substances de formule générale (I) 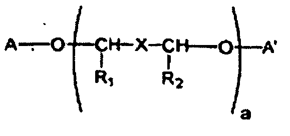
dans laquelle A et A' sont des radicaux organiques identiques ou différents sélectionnés dans l'ensemble des radicaux alkyle, acyle et hydroxyacyle ramifiés et non ramifiés, saturés et insaturés, qui comptent de 10 à 30 atomes de carbone, et en outre dans l'ensemble des groupes hydroxyacyle liés les uns aux autres par des fonctions ester selon le schéma: 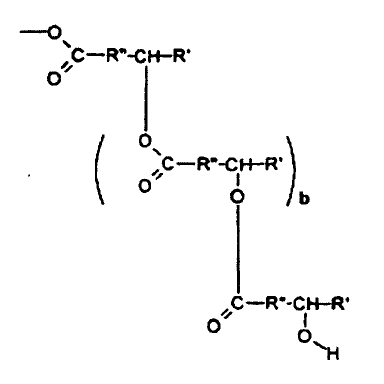
dans lequel R' est sélectionné dans l'ensemble des groupes alkyle ramifiés et non ramifiés qui comptent de 1 à 20 atomes de carbone et R" est sélectionné dans l'ensemble constitué des groupes alkylène ramifiés et non ramifiés qui comptent de 1 à 20 atomes de carbone, b étant un nombre de 0 à 200,
- a étant un nombre de 1 à 100, de préférence de 2 à 60 et en particulier de 5 à 40,
- X est une liaison simple ou le groupe: 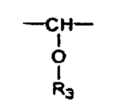
- R₁ et R₂ sont sélectionnés indépendamment l'un de l'autre dans l'ensemble constitué de H et du méthyle,
- R₃ est sélectionné dans l'ensemble constitué de H ainsi que des radicaux alkyle et acyle ramifiés et non ramifiés, saturés et insaturés, qui comptent de 1 à 20 atomes de carbone

2. Emulsions selon la revendication 1, **caractérisées en ce que** leur teneur en eau et en substances solubles dans l'eau est supérieure à 80% en poids et en particulier supérieure à 85% en poids, chaque fois par rapport au poids total des préparations.

3. Emulsions selon la revendication 1, **caractérisées en ce que** comme substance tensioactive, on sélectionne le dipolyhydroxystéarate de polyéthylèneglycol-30.

4. Emulsions selon la revendication 1, **caractérisées en ce que** la phase huileuse est constituée pour au moins 50% en poids et de préférence pour plus de 75% en poids d'au moins une substance sélectionnée dans l'ensemble constitué de la vaseline (petrolatum), de l'huile de paraffine et des polyoléfines et parmi ces dernières, de préférence du polydécène.
